# EUROPEAN PATENT APPLICATION

(11) **EP 2 859 889 A1**
(43) Date of publication of application: **15.04.2015**
(21) Application number: 13382406.0
(22) Date of filing: 14.10.2013
(51) Int. Cl.: A61K 31/14, A61K 31/191, A61K 31/405, A61K 31/685, A61K 33/06, A61P 25/26, A61P 25/28

(54) **Compositions and methods for improving cognitive function**

(71) Applicant: ABBOTT LABORATORIES, Abbott Park, IL 60064-3500 (US)
(72) Inventor: Barranco Perez, Alejandro, 18110 Las Gabias (Granada) (ES); Vazquez Hernandez, Enrique, 18151 Ogijares (Granada) (ES); Ramirez Gonzalez, Maria, 18003 Granada (ES); Rueda Cabrera, Ricardo, 18008 Granada (ES)
(74) Representative: Ungria López, Javier

(57) **Abstract**

Compositions including a combination of a source of choline, a source of magnesium, and a source of L-tryptophan are provided for improving cognitive performance in a subject. The compositions may improve a cognitive impairment and/or brain dysfunction associated with age-related cognitive decline or cognitive decline resulting from a neurodegenerative disease. The compositions may also be effective in improving memory acquisition, memory retention, and recall.

## Description

### FIELD OF THE DISCLOSURE

The present disclosure relates to compositions for improving cognitive performance and/or memory, including memory acquisition and memory retention and recall, in an individual, as well as reducing brain dysfunction and cognitive decline, and to methods of administering the compositions to a subject. In particular, the compositions comprise a source of choline, a source of magnesium, and a source of L-tryptophan.

### BACKGROUND OF THE DISCLOSURE

Nutritional powders and liquids comprising a targeted selection of nutritional ingredients are well known and widely available, some of which may provide a complete source of nutrition while others may provide a supplemental source. These nutritionals include powders that can be reconstituted with water or another aqueous liquid, as well as ready to drink nutritional liquids such as milk or protein based emulsions or non-emulsified liquids. These nutritional liquids are especially useful when formulated with selected nutritional ingredients.

An estimated 4 to 5 million Americans, about 15% of those older than age 65, have some form and degree of cognitive failure. Cognitive failure, which includes dysfunction or loss of cognitive function (e.g., learning, thinking, memory), commonly occurs in association with central nervous system ("CNS") disorders or conditions, including neurodegenerative diseases. Cognitive dysfunction may cause significant impairment of social and/or occupational functioning, which can interfere with the ability of an individual to perform activities of daily living and greatly impact the autonomy and quality of life of the individual.

Accordingly, there has recently been an increased interest in designing and marketing so-called "smart formulations" that include nutritional products designed specifically for brain health and nourishment. Many of these products are specifically designed for improving cognition and preventing neurodegenerative diseases and related cognitive decline. To date, these formulations and products have had limited success.

As such, there is a need for compositions and methods for enhancing cognitive performance, and particularly, memory acquisition and memory recall, that may contribute to the learning and memory processes. It would be beneficial if such compositions and methods could also improve a cognitive impairment and/or brain dysfunction, such as from age-related cognitive decline or from neurodegenerative diseases.

### SUMMARY OF THE DISCLOSURE

In a first exemplary embodiment, the present disclosure is directed to a composition suitable for maintaining or improving cognition in a subject. The composition comprises a source of choline in an amount sufficient to provide 0.4 to 8 grams of choline per day; a source of magnesium in an amount sufficient to provide 0.2 to 3 grams of magnesium per day; and a source of L-tryptophan.

In a second exemplary embodiment, the present disclosure is directed to a composition suitable for maintaining or improving cognition in a subject. The composition comprises 0.1 to 40 weight percent of a source of choline; 0.1 to 30 weight percent of a source of magnesium; and a source of L-tryptophan. The weight percentages are of the total solids of the composition (i.e., excluding water, solvents, and/or inert fillers).

In a third exemplary embodiment, the present disclosure is directed to a method of maintaining or improving cognition in a subject. The method comprises administering to the subject an effective amount of a composition. The composition comprises a source of choline, a source of magnesium, and a source of L-tryptophan. The effective amount of the composition maintains or stimulates brain function in the subject and thereby maintains or improves cognition of the subject.

The third embodiment also provides a composition for use in maintaining or improving cognition in a subject, the composition comprising a source of choline, a source of magnesium, and a source of L-tryptophan. An effective amount of the composition maintains or stimulates brain function in the subject and thereby maintains or improves cognition of the subject.

In addition the third embodiment provides the use of a composition in the manufacture of a medicament for maintaining or improving cognition wherein the composition comprises a source of choline, a source of magnesium and a source of L-tryptophan. An effective amount of the composition maintains or stimulates brain function in a subject and thereby maintains or improves cognition of the subject.

In a fourth exemplary embodiment, the present disclosure is directed to a composition suitable for maintaining or improving cognition in a subject. The composition comprises 0.1 to 40 weight percent glycerophosphocholine; 0.1 to 30 weight percent magnesium threonate; and L-tryptophan. The weight percentages are of the total solids of the composition (i.e., excluding water, solvents, and/or inert fillers).

In a fifth embodiment, the present disclosure is directed to a composition comprising a source of choline in an amount sufficient to provide 0.4 to 8 grams of choline per day; a source of magnesium in an amount sufficient to provide 0.2 to 3 grams of magnesium per day; and a source of L-tryptophan for use in the treatment of the human or animal body by therapy. The present disclosure is also directed to a composition comprising 0.1 to 40 weight percent of a source of choline; 0.1 to 30 weight percent of a source of magnesium; and a source of L-tryptophan for use in the treatment of the human or animal body by therapy. The present disclosure if also directed to a composition comprising 0.1 to 40 weight percent glycerophosphocholine; 0.1 to 30 weight percent magnesium threonate; and L-tryptophan. These compositions are for use in maintaining or improving cognition. The weight percentages are of the total solids of the composition (i.e., excluding water, solvents, and/or inert fillers).

### BRIEF DESCRIPTION OF THE DRAWINGS

Various aspects and advantages of the inventive concepts will become more readily apparent to those of ordinary skill in the art after reviewing the following detailed description and accompanying drawings, wherein:
FIG. 1a is a bar graph illustrating the mean number of visits to the corners for each group of mice during the "shuttle" test of the Example;
FIG. 1b is a bar graph illustrating the mean number of visits to the corners for each group of mice during the "reverse shuttle" test of the Example;
FIG. 2a is a bar graph illustrating the percentage of correct visits to the reward corners for each group of mice during the "shuttle" test of the Example; and
FIG. 2b is a bar graph illustrating the percentage of correct visits to the reward corners for each group of mice during the "reverse shuttle" test of the Example.

### DETAILED DESCRIPTION

While embodiments encompassing the general inventive concepts may take various forms, there will hereinafter be described various embodiments with the understanding that the present disclosure is to be considered merely an exemplification, and the general inventive concepts are not intended to be limited to the disclosed embodiments.

The compositions and methods herein are directed to compositions comprising a source of choline, a source of magnesium, and a source of L-tryptophan that can improve general cognitive performance in a subject as defined herein, including memory acquisition, memory retention, and memory recall. These and other essential and optional elements and features of the various embodiments are described in detail hereafter.

The term "subject" as used herein, unless otherwise specified, refers to a living mammal.

The term "older adult" as used herein, unless otherwise specified, refers to an individual of at least 45 years of age, including at least 50 years of age, including at least 55 years of age, including at least 60 years of age, including at least 65 years of age, including at least 70 years of age, including at least 75 years of age, including at least 80 years of age, further including from about 55 years of age to about 80 years of age.

The term "choline" as used herein, unless otherwise specified, refers to choline and derivatives and analogs thereof.

The term "glycerophosphocholine" as used herein, unless otherwise specified, refers to glycerophosphocholine and derivatives and analogs thereof.

The term "magnesium citrate" as used herein, unless otherwise specified, refers to magnesium citrate and derivatives and analogs thereof.

The term "magnesium chloride" as used herein, unless otherwise specified, refers to magnesium chloride and derivatives and analogs thereof.

The term "magnesium threonate" as used herein, unless otherwise specified, refers to magnesium threonate and derivatives and analogs thereof.

The term "L-tryptophan" as used herein, unless otherwise specified, refers to L-tryptophan and derivatives and analogs thereof.

The term "bioavailable" as used herein, unless otherwise specified, refers to the ability of a compound to enter into and remain in the bloodstream of an individual such that the substance can be absorbed into cells in the body. As the degree of bioavailability of a compound increases, the compound becomes more likely to enter into and remain in the bloodstream where it can be absorbed and used by the body. As the degree of bioavailability of a compound decreases, the compound becomes more likely to go directly through the gastrointestinal area and be expelled from the body before entering the bloodstream. For example, the amount of magnesium to reach the brain is generally higher when magnesium threonate is used as a source of magnesium instead of other common sources of magnesium (e.g., magnesium citrate, magnesium chloride).

The term "nutritional composition" as used herein, unless otherwise specified, refers to nutritional powders, solids, semi-solids, liquids, and semi-liquids that comprise at least one of protein, carbohydrate, and lipid and are suitable for oral administration to a subject. The nutritional composition may further comprise vitamins, minerals, and other ingredients and represent a sole, primary, or supplemental source of nutrition.

The terms "powder," "reconstitutable," or "reconstitutable powder" as used herein, unless otherwise specified, each describe a physical form of a composition, or portion thereof, that is flowable or scoopable and can be reconstituted with water or another aqueous liquid prior to consumption.

The terms "liquid nutritional composition" and "nutritional liquid" are used interchangeably herein, and unless otherwise specified, refer to nutritional products in ready-to-consume liquid form and/or concentrated liquid form.

The terms "fat," "lipid," and "oil" as used herein, unless otherwise specified, are used interchangeably to refer to lipid materials derived or processed from plants and/or animals. These terms also include synthetic lipid materials so long as such synthetic materials are suitable for administration to subjects as defined herein.

The term "serving" as used herein, unless otherwise specified, is intended to be construed as any amount that is intended to be consumed by an individual in one sitting or within one hour or less. The size of a serving (i.e., "serving size") may be different from subject to subject depending on one or more of several factors including, but not limited to, age, body mass, gender, race, genus/species, and/or health. For a typical adult human, an average serving size of the compositions disclosed herein is one that provides from 0.4 to 8 grams of choline per day, from 0.2 to 3 grams of magnesium per day, and an amount of a source of L-tryptophan. In certain embodiments of the compositions, the compositions have a weight ratio of L-tryptophan-to-total large neutral amino acids ranging from 0.2 to 0.5.

The term "susceptible" as used herein, unless otherwise specified, means having little resistance to a certain condition or disease, including being genetically predisposed, having a family history of, and/or having symptoms of the condition or disease.

The term "synergy," "synergistic amount," and "synergistic effect" as used herein, unless otherwise specified, refers to the interaction of two or more compounds so that their combined effect is greater than the additive sum of their individual effects.

The term "cognitive performance" as used herein, unless otherwise specified, refers to the learning, thinking, and memory functions (i.e., memory acquisition, memory retention, and memory recall) of the mammalian brain. Accordingly, the term "improving cognitive performance" as used herein, unless otherwise specified, refers to improving the learning, thinking, and/or memory (memory acquisition, memory retention and memory recall) functions of an individual.

The term "improving a cognitive impairment and/or brain dysfunction" as used herein, unless otherwise specified, refers to treating, preventing, and/or reducing the incidence or severity of cognitive decline associated with agerelated cognitive decline or neurodegenerative disease.

The term "age-related cognitive decline" as used herein, unless otherwise specified, refers to a gradual decline in cognitive performance that is a normal consequence of aging.

The term "neurodegenerative disease" as used herein, unless otherwise specified, refers to the progressive loss of structure or function of neurons, including the death of neurons, and includes but is not limited to diseases such as Parkinson's disease, Alzheimer's disease, Huntington's disease, dementia, amyotrophic lateral sclerosis, stroke, and schizophrenia.

The term "regular intervals" as used herein, unless otherwise specified, refers to administration in a repeating, periodic fashion where the time between administrations is approximately (or at least intended to be approximately) the same.

The term "readily dispersible" as used herein, unless otherwise specified, refers to a solid that is easily blended with a liquid such that little to no agglomeration or settling occurs with normal stirring for one minute. "Readily dispersible" is broader in scope than "dissolving."

The term "large neutral amino acids" as used herein, unless otherwise specified, refers to L-valine, L-tyrosine, L-phenylalanine, L-leucine, and L-isoleucine, whether free amino acids or as part of a protein, that are transported to the brain in the same manner as L-tryptophan, and therefore compete with L-tryptophan for biochemical entry into the brain. Therefore, the "weight ratio of L-tryptophan-to-total large neutral amino acids" is a ratio of the weight amount of tryptophan divided by the sum weight amounts of L-valine, L-tyrosine, L-phenylalanine, L-leucine, and L-isoleucine present in a particular composition.

All percentages, parts and ratios as used herein, are by weight of the total product, unless specified otherwise. All such weights as they pertain to listed ingredients are based on the active level and, therefore, do not include solvents or by-products that may be included in commercially available materials, unless specified otherwise.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

All ranges and parameters, including but not limited to percentages, parts, and ratios, disclosed herein are understood to encompass any and all sub-ranges assumed and subsumed therein, and every number between the endpoints. For example, a stated range of "1 to 10" should be considered to include any and all subranges between (and inclusive of) the minimum value of 1 and the maximum value of 10; that is, all subranges beginning with a minimum value of 1 or more (e.g., 1 to 6.1) and ending with a maximum value of 10 or less (e.g., 2.3 to 9.4, 3 to 8, 4 to 7), and finally to each number 1, 2, 3, 4, 5, 6, 7, 8, 9, and 10 contained within the range.

The various embodiments of the compositions of the present disclosure may also be substantially free of any optional ingredient or feature described herein, provided that the remaining composition still contains all of the required ingredients or features as described herein. In this context, and unless otherwise specified, the term "substantially free" means that the selected composition contains less than a functional amount of the optional ingredient, typically less than about 1%, including less than about 0.5%, including less than about 0.1%, and also including zero percent, by weight of such optional ingredient.

The compositions may comprise, consist of, or consist essentially of the required elements of the products as described herein, as well as any additional or optional element described herein or otherwise useful in product applications.

In a first exemplary embodiment, the present disclosure is directed to a composition suitable for maintaining or improving cognition in a subject. The composition comprises a source of choline in an amount sufficient to provide 0.4 to 8 grams of choline per day; a source of magnesium in an amount sufficient to provide 0.2 to 3 grams of magnesium per day; and a source of L-tryptophan.

In a second exemplary embodiment, the present disclosure is directed to a composition suitable for maintaining or improving cognition in a subject. The composition comprises 0.1 to 40 weight percent of a source of choline; 0.1 to 30 weight percent of a source of magnesium; and a source of L-tryptophan. The weight percentages are of the total solids of the composition (i.e., excluding water, solvents, and/or inert fillers).

In a third exemplary embodiment, the present disclosure is directed to a method of maintaining or improving cognition in a subject. The method comprises administering to the subject an effective amount of a composition. The composition comprises a source of choline, a source of magnesium, and a source of L-tryptophan. The effective amount of the composition maintains or stimulates brain function in the subject and thereby maintains or improves cognition of the subject.

The third embodiment also provides a composition for use in maintaining or improving cognition in a subject, the composition comprising a source of choline, a source of magnesium, and a source of L-tryptophan. An effective amount of the composition maintains or stimulates brain function in the subject and thereby maintains or improves cognition of the subject.

In addition the third embodiment provides the use of a composition in the manufacture of a medicament for maintaining or improving cognition wherein the composition comprises a source of choline, a source of magnesium and a source of L-tryptophan. An effective amount of the composition maintains or stimulates brain function in a subject and thereby maintains or improves cognition of the subject.

In a fourth exemplary embodiment, the present disclosure is directed to a composition suitable for maintaining or improving cognition in a subject. The composition comprises 0.1 to 40 weight percent glycerophosphocholine; 0.1 to 30 weight percent magnesium threonate; and L-tryptophan. The weight percentages are of the total solids of the composition (i.e., excluding water, solvents, and/or inert fillers).

In a fifth embodiment, the present disclosure is directed to a composition comprising a source of choline in an amount sufficient to provide 0.4 to 8 grams of choline per day; a source of magnesium in an amount sufficient to provide 0.2 to 3 grams of magnesium per day; and a source of L-tryptophan for use in the treatment of the human or animal body by therapy. The present disclosure is also directed to a composition comprising 0.1 to 40 weight percent of a source of choline; 0.1 to 30 weight percent of a source of magnesium; and a source of L-tryptophan for use in the treatment of the human or animal body by therapy. The present disclosure if also directed to a composition comprising 0.1 to 40 weight percent glycerophosphocholine; 0.1 to 30 weight percent magnesium threonate; and L-tryptophan. These compositions are for use in maintaining or improving cognition. The weight percentages are of the total solids of the composition (i.e., excluding water, solvents, and/or inert fillers).

The compositions of the present disclosure each comprise the combination of a source of choline (e.g., glycerophosphocholine), a source of magnesium (e.g., magnesium threonate), and a source of L-tryptophan, such that the compositions provide synergistic benefits in improving cognitive performance in a subject that consumes one or more of the compositions.

In certain embodiments of the first, second, third and fourth exemplary embodiments, the composition further comprises at least one ingredient selected from the group consisting of: a protein, a carbohydrate, a lipid, a stabilizer, a vitamin, a mineral, a flavoring agent, a masking agent, an emulsifier, and combinations thereof. These and other optional ingredients are described in further detail herein.

### Product Form

The compositions of the present disclosure may be formulated and administered in any known or otherwise suitable product form. Any powder, solid, semi-solid, liquid, or semi-liquid form, including combinations or variations thereof, are suitable for use herein, provided that such forms allow for safe and effective delivery of the essential ingredients disclosed herein to the subject. If present in the powder form, the composition may be reconstitutable in a consumable aqueous liquid. Exemplary embodiments of consumable aqueous liquids include (potable) water, milk, juice, and so forth. If present in the liquid or semi-liquid form, the composition may further comprise water. If present in the solid form, the composition may take the form of, for example, a pill, a capsule, a tablet, a chewable solid (e.g., a chewable tablet, a nutritional bar, etc.), or any other orally administered solid form.

The compositions are suitably formed as aqueous emulsions, including water-in-oil emulsions, oil-in-water emulsions, or complex (e.g., oil-in-water-in-oil) emulsions, or other emulsion systems. As applied to the compositions herein, the emulsion embodiments are typically oil-in-water emulsions comprising an internal or discontinuous oil phase that comprises the source of choline, the source of magnesium, and the source of L-tryptophan as defined herein.

### Source of Choline

In certain embodiments of the first, second, third, and fourth exemplary embodiments, the composition comprises a source of choline. In certain embodiments, the source of choline is present in the composition in an amount sufficient to provide 0.4 to 8 grams of choline per day. In certain embodiments, the composition comprises 0.1 to 40 weight percent of a source of choline. The source of choline can be derived from any known or otherwise suitable source of choline, including mammalian milk. While not wishing to be bound by theory, it is believed that choline is important for the structural integrity of cell membranes, cholinergic neurotransmission, transmembrane signaling, methyl metabolism, and lipid-cholesterol transport and metabolism in mammals.

In certain embodiments, the source of choline is selected from the group consisting of choline, phosphocholine, phosphatidyl-choline, glycerophosphocholine, and combinations thereof. In certain embodiments, the source of choline is glycerophosphocholine.

The source of choline may comprise from at least about 0.1 weight percent, at least about 0.2 weight percent, at least about 0.3 weight percent, at least about 0.5 weight percent, at least about 1 weight percent, at least about 2 weight percent, at least about 5 weight percent, or at least about 8 weight percent; and up to about 10 weight percent, up to about 12 weight percent, up to about 15 weight percent, up to about 20 weight percent, up to about 30 weight percent, or up to about 40 weight percent, of the composition, wherein the weight percentages are based on the total solids of the composition.

In certain embodiments of the composition, the source of choline is present in an amount sufficient to provide about 0.4 to about 8 grams of choline per day to a subject that consumes the composition. The composition may provide at least about 0.4 gram, at least about 0.5 gram, at least about 0.6 gram, at least about 0.8 gram, at least about 1 gram, at least about 1.2 grams, at least about 1.5 grams, at least about 2 grams, or at least about 3 grams; and up to about 3.5 grams, up to about 4 grams, up to about 4.5 grams, up to about 5 grams, up to about 5.5 grams, up to about 6 grams, up to about 6.5 grams, up to about 7 grams, up to about 7.5 grams, or up to about 8 grams of choline per day to a subject that consumes the composition.

Glycerophosphocholine is a nutrient present in all mammalian cells. Generally, relative to other common sources of choline, glycerophosphocholine is a rapidly absorbed source of choline that is believed to more easily enter the brain than other common sources of choline. In certain embodiments of the first, second, third, and fourth exemplary embodiments, the source of choline is glycerophosphocholine.

### Source of Magnesium and Source of Threonate

Magnesium, the fourth most abundant ion in the body and a cofactor for more than three hundred enzymes, is essential for the proper functioning of many tissues and organs in mammals, including tissues and organs of the cardiovascular, neuromuscular, and nervous systems. The mammalian brain requires magnesium to modulate the voltage-dependent block of N-methyl-D-aspartate ("NMDA") receptors, controlling their opening during coincidence detection that is critical for synaptic plasticity and cognitive function.

In certain embodiments of the first, second, third, and fourth exemplary embodiments, the composition comprises a source of magnesium. The source of magnesium may be selected from the group consisting of magnesium citrate, magnesium chloride, magnesium threonate, and combinations thereof. In certain embodiments, the source of magnesium is magnesium threonate.

The source of magnesium may comprise from at least about 0.1 weight percent, at least about 0.2 weight percent, at least about 0.3 weight percent, at least about 0.5 weight percent, at least about 1 weight percent, at least about 2 weight percent, at least about 5 weight percent, or at least about 8 weight percent; and up to about 10 weight percent, up to about 12 weight percent, up to about 15 weight percent, up to about 20 weight percent, up to about 25 weight percent, or up to about 30 weight percent, of the composition, wherein the weight percentages are based on the total solids of the composition.

In certain embodiments of the composition, the source of magnesium is present in an amount sufficient to provide about 0.2 to about 3 grams of magnesium per day to a subject that consumes the composition. The composition may provide at least about 0.3 gram, at least about 0.4 gram, at least about 0.5 gram, at least about 0.6 gram, at least about 0.8 gram, at least about 1 gram, at least about 1.2 grams, at least about 1.4 grams, or at least about 1.5 grams; and up to about 2 grams, up to about 2.2 grams, up to about 2.4 grams, up to about 2.7 grams, or up to about 3 grams of magnesium per day to a subject that consumes the composition.

In certain embodiments of the composition, the composition further comprises a source of threonate. In certain embodiments, the source of magnesium and the source of threonate are magnesium threonate, or the source of magnesium and the source of threonate may comprise at least two compounds. In certain embodiments comprising a source of threonate, the source of threonate is present in the composition in an amount sufficient to provide 0.2 to 3 grams of threonate per day. Exemplary sources of threonate include, but are not limited to, threonic acid, L-threonate, calcium threonate, magnesium threonate, and combinations thereof. Furthermore, the term "magnesium threonate" expressly includes "magnesium L-threonate." Magnesium threonate is a composition having a relatively high bioavailability compared to other magnesium-containing compositions that may be utilized in nutritional compositions (e.g., magnesium citrate, magnesium chloride). As a result of its relatively high bioavailability, magnesium threonate is particularly suitable for use in the compositions disclosed herein, which, when administered to a subject, can thereby lead to maintained or improved cognition of the subject.

In certain embodiments of the composition comprising magnesium threonate, the magnesium threonate may comprise from at least about 0.1 weight percent, at least about 0.2 weight percent, at least about 0.3 weight percent, at least about 0.5 weight percent, at least about 1 weight percent, at least about 2 weight percent, at least about 5 weight percent, or at least about 8 weight percent; and up to about 10 weight percent, up to about 12 weight percent, up to about 15 weight percent, up to about 20 weight percent, up to about 25 weight percent, or up to about 30 weight percent, of the composition, wherein the weight percentages are based on the total solids of the composition.

Furthermore, in certain embodiments of the composition comprising magnesium threonate, the magnesium threonate is present in an amount sufficient to provide about 0.5 to about 5 grams of magnesium threonate per day to a subject that consumes the composition. The composition may provide at least about 0.5 gram, at least about 0.6 gram, at least about 0.7 gram, at least about 0.8 gram, at least about 1 gram, at least about 1.2 grams, at least about 1.5 grams, at least about 2 grams, or at least about 2.5 grams; and up to about 3 grams, up to about 3.5 grams, up to about 4 grams, up to about 4.5 grams, or up to about 5 grams of magnesium threonate per day to a subject that consumes the composition.

### Source of L-Tryptophan and Optionally Large Neutral Amino Acids

In certain embodiments of the first, second, third, and fourth exemplary embodiments, the composition comprises L-tryptophan, or a source thereof. The L-tryptophan can be derived from any known or otherwise suitable source of L-tryptophan. One exemplary source of L-tryptophan suitable for use in the embodiments of the present disclosure is alpha-lactalbumin protein.

Brain serotonin ("5-HT") receptors are widely distributed in the central nervous system of mammals and, while not wishing to be bound by theory, are believed to involve the regulation of several behavioral and physiological functions (e.g., mood, sleep, appetite, sexual behavior, and possibly others). Reduced 5-HT function is particularly recognized as a vulnerability factor involved in cognitive deficit. Because 5-HT is synthesized from the essential amino acid L-tryptophan, dietary manipulation of L-tryptophan concentration in blood plasma may influence brain 5-HT synthesis. An increase in the intake of L-tryptophan can lead to an increased amount and/or concentration of 5-HT in the brain, thereby allowing for maintaining or improving cognition in a subject.

L-tryptophan may comprise from at least about 0.1 weight percent, at least about 0.2 weight percent, at least about 0.3 weight percent, at least about 0.5 weight percent, at least about 1 weight percent, at least about 2 weight percent, at least about 5 weight percent, or at least about 8 weight percent, and up to about 10 weight percent of the composition, wherein the weight percentages are based on the total solids of the composition.

In certain embodiments of the composition, the L-tryptophan or source thereof is present in an amount sufficient to provide about 0.5 to about 5 grams of L-tryptophan per day to a subject that consumes the composition. The composition may provide at least about 0.5 gram, at least about 0.6 gram, at least about 0.7 gram, at least about 0.8 gram, at least about 1 gram, at least about 1.2 grams, at least about 1.5 grams, at least about 2 grams, or at least about 2.5 grams; and up to about 3 grams, up to about 3.5 grams, up to about 4 grams, up to about 4.5 grams, or up to about 5 grams of L-tryptophan per day to a subject that consumes the composition.

In certain embodiments of the first, second, third, and fourth exemplary embodiment, the composition further comprises at least one large neutral amino acid. When present, the amount of L-tryptophan and the total amount of large neutral amino acids are present in the composition at a weight ratio of L-tryptophan-to-total large neutral amino acids ranging from 0.2 to 0.5. In certain embodiments, the weight ratio of L-tryptophan-to-total large neutral amino acids in the composition is at least 0.2. The weight ratio may be at least 0.21, or at least 0.22, or at least 0.23, or at least 0.24, or at least 0.25, or at least 0.3, or at least 0.4, up to 0.5.

### Macronutrients

The compositions of the present disclosure may further comprise one or more optional macronutrients in addition to the source of choline, the source of magnesium, and the source of L-tryptophan described herein. The optional macronutrients include proteins, carbohydrates, lipids, and combinations thereof. The compositions are desirably formulated as dietary products containing one or more macronutrients. In other words, the compositions are desirably formulated as nutritional compositions as defined herein.

Macronutrients suitable for use herein include any protein, carbohydrate, or lipid, or source thereof, that is known for or otherwise suitable for use in an oral nutritional composition, provided that the optional macronutrient is safe and effective for oral administration and is otherwise compatible with the other ingredients in the composition.

The concentration or amount of optional carbohydrate, protein, and/or lipid in nutritional compositions can vary considerably depending upon the particular product form (e.g., nutritional bars or other solid dosage forms; milk or soy-based liquids; clear beverages; reconstitutable powders) and the various other targeted dietary needs. These optional macronutrients are most typically formulated within any of the embodied ranges described in Tables 1A and 1 B below, keeping in mind that "protein" refers to proteins as defined herein and does not include L-tryptophan, which is separately defined. Furthermore, depending on the embodiment, the values for "protein" may be further restricted according to the ratio of L-tryptophan-to-total large neutral amino acid defined herein.

**Table 1A**

| Nutrient (% total calories) | Example A | Example B | Example C |
|---|---|---|---|
| Protein | 0-100 | 5-40 | 15-25 |
| Carbohydrate | 0-100 | 10-70 | 40-50 |
| Lipid | 0-100 | 20-65 | 35-55 |

| | | | |
|---|---|---|---|
| Each numerical value preceded by the term "about" | | | |

**Table 1B**

| Nutrient (wt% composition) | Example D | Example E | Example F |
|---|---|---|---|
| Protein | 0-98 | 1-30 | 2-10 |
| Carbohydrate | 0-98 | 1-50 | 10-30 |
| Lipid | 0-98 | 1-30 | 3-15 |

| | | | |
|---|---|---|---|
| Each numerical value preceded by the term "about" | | | |

### Protein

Optional proteins suitable for use in nutritional compositions include hydrolyzed, partially hydrolyzed, or non-hydrolyzed proteins or protein sources, and can be derived from any known or otherwise suitable source such as milk (e.g., casein, whey), animal (e.g., meat, fish, egg albumen, insect, earthworm), cereal (e.g., rice, corn), vegetable (e.g., soy, pea, potato), or combinations thereof. The proteins for use herein can also include, or be entirely or partially replaced by, free amino acids known for use in nutritional products, non-limiting examples of which include L-glutamine, L-tyrosine, L-methionine, L-cysteine, taurine, L-arginine, L-carnitine, and combinations thereof. In certain embodiments, the free amino acids are large neutral amino acids as described herein.

### Carbohydrate

Optional carbohydrates suitable for use in nutritional compositions may be simple, complex, variations or combinations thereof. Non-limiting examples of suitable carbohydrates include hydrolyzed or modified starch or cornstarch, maltodextrin, isomaltulose, sucromalt, glucose polymers, sucrose, corn syrup, corn syrup solids, rice-derived carbohydrate, glucose, fructose, lactose, high fructose corn syrup, honey, sugar alcohols (e.g., maltitol, erythritol, sorbitol), artificial sweeteners (e.g., sucralose, acesulfame potassium, stevia), and combinations thereof.

Optional carbohydrates suitable for use herein also include soluble dietary fiber, non-limiting examples of which include gum Arabic, fructooligosaccharides ("FOS"), sodium carboxymethyl cellulose, guar gum, citrus pectin, low and high methoxy pectin, oat and barley glucans, carrageenan, psyllium, and combinations thereof. Insoluble dietary fiber is also suitable as a carbohydrate source herein, non-limiting examples of which include oat hull fiber, pea hull fiber, soy hull fiber, soy cotyledon fiber, sugar beet fiber, cellulose, corn bran, and combinations thereof.

### Lipid

In addition to the ingredients described above, nutritional compositions may further comprise lipid or a source thereof, most typically as emulsified fat. Suitable fats or sources thereof for use herein include any fat or fat source that is suitable for use in an oral nutritional product and is compatible with the elements and features of such products. Desirably, a fat source will provide at least one long chain polyunsaturated acid ("LC-PUFA") such as docosahexanoic acid ("DHA"), arachidonic acid ("ARA"), and/or eicosapentaenoic acid ("EPA"), although these LC-PUFAs may be optionally added to nutritional compositions outside of, or in addition to, a fat source.

Optional lipids suitable for use in nutritional compositions include coconut oil, fractionated coconut oil, soy oil, corn oil, olive oil, safflower oil, high oleic safflower oil, high GLA-safflower oil, medium chain triglycerides ("MCT oil"), sunflower oil, high oleic sunflower oil, palm and palm kernel oils, palm olein, canola oil, flaxseed oil, borage oil, cottonseed oils, evening primrose oil, blackcurrant seed oil, transgenic oil sources, fungal oils, marine oils (e.g., tuna, sardine), and combinations thereof.

### Other Optional Ingredients

The compositions described herein may further comprise other optional ingredients that may modify the physical, chemical, hedonic or processing characteristics of the products or serve as pharmaceutical or additional nutritional components when used in the targeted population. Many such optional ingredients are known or otherwise suitable for use in nutritional compositions and may also be used in the compositions described herein, provided that such optional ingredients are safe and effective for administration and are compatible with the essential and other ingredients in the selected product form.

Non-limiting examples of such optional ingredients include preservatives, antioxidants, emulsifying agents, buffers, pharmaceutical actives, additional nutrients as described herein, colorants, flavoring agents, masking agents, thickeners (e.g., induced viscosity fibers), emulsifiers (e.g., lecithin), additional stabilizers, cereal beta-glucans (barley beta-glucan), probiotics (e.g., Lactobacillus rhamnosus HN001 (DR20)), prebiotics (fructooligosaccharides, galactooligosaccharides, inulin, oligofructose), Salacia extract, and so forth.

In certain exemplary embodiments, the composition can further comprise a flavoring agent. For example, the composition may comprise up to 2% by weight of a flavoring agent. Suitable flavoring agents for use with the exemplary embodiments of the composition include, for example, vanilla, cocoa, vanillin, salt, coffee, chocolate flavoring, berry flavors, and fruit flavors, acids (e.g., lactic, malic), caramel, mint, natural and/or artificial sweeteners, sodium sources such as sodium chloride, hydrocolloids, and combinations thereof.

The compositions may further comprise vitamins or related nutrients, non-limiting examples of which include carotenoids (e.g., beta-carotene, zeaxnthin, lutein, lycopene), biotin, inositol, folic acid, pantothenic acid, TPAN, choline, Vitamin A, thiamine, riboflavin, niacin, pyridoxine, cyanocobalamin, ascorbic acid, Vitamin D, Vitamin E, Vitamin K, and various salts, esters, or other derivatives thereof, and combinations thereof.

The compositions may further comprise minerals, non-limiting examples of which include phosphorus, magnesium, calcium, iron, zinc, manganese, copper, sodium, potassium, molybdenum, chromium, selenium, chloride, and combinations thereof.

In some embodiments disclosed herein, the composition may comprise a stabilizer. Any stabilizer that is known or otherwise suitable for use in nutritional compositions is also suitable for use herein, some non-limiting examples of which include gums such as xanthan gum. In certain embodiments according to the first, second, third, and fourth exemplary embodiments disclosed herein, the stabilizer may represent from about 0.1% to about 5%, from about 0.5% to about 3%, or from about 0.7% to about 1.5% by weight of the composition.

In some embodiments disclosed herein, the compositions optionally include one or more masking agents to reduce or otherwise obscure the development of any residual bitter flavors and after taste in the compositions over time. Suitable masking agents include natural and artificial sweeteners, sodium sources such as sodium chloride, and hydrocolloids, such as guar gum, xanthan gum, carrageenan, gellan gum, and combinations thereof. The amount of masking agent in certain embodiments of the composition may vary depending upon the particular masking agent selected, other ingredients in the formulation, and other formulation or product target variables. Such amounts, however, most typically range from 0.1% to 3%, from 0.15% to 3%, or from 0.2% to 2.5% by weight of the composition.

In some embodiments, the compositions may include fish oil masking agents to mask "fishy" type flavors/aroma notes that commonly occur with the presence of fish/marine oil. For example, in some embodiments, the composition may include the lipid-amylose complex described herein. Specifically, the combination of monoglycerides and low DE glucose polymers, such as DE-1 maltodextrin, form a lipid-amylose complex that can bind to oxidation products such as the fatty acid chain of aldehydes or ketones formed during oxidation of marine oils.

### Methods of Manufacture

The compositions may be prepared by any known or otherwise effective manufacturing technique for preparing the selected product form. Many such techniques are known for any given product form such as nutritional liquids and nutritional powders and can easily be applied by one of ordinary skill in the nutrition and formulation arts to the compositions described herein.

Liquid, milk or soy-based nutritional liquids, for example, may be prepared by first forming an oil and fiber blend containing all formulation oils, any emulsifier, fiber and fat-soluble vitamins. Additional slurries (typically a carbohydrate slurry and at least one protein slurry) are prepared separately by mixing the carbohydrate and minerals together and the protein in water. The slurries are then mixed together with the oil blend. The resulting mixture is homogenized, heat processed, standardized with any water-soluble vitamins, flavored and the liquid terminally sterilized or aseptically filled or dried to produce a powder.

The compositions of the present disclosure may also be manufactured by other known or otherwise suitable techniques not specifically described herein without departing from the spirit and scope of the present disclosure. The present embodiments are, therefore, to be considered in all respects as illustrative and not restrictive and that all changes and equivalents also come within the description of the present disclosure.

### Methods of Use

Though administration of the composition may occur in any conceivable fashion known in the art, the methods of administering the compositions described herein typically include oral administration to a subject to improve cognitive performance of the subject. In certain embodiments of the methods, the subject is a human. Particularly, the combination of a source of choline (e.g., glycerophosphocholine), a source of magnesium (e.g., magnesium threonate), and a source of L-tryptophan may improve general cognition by producing a sequential action on memory acquisition, memory retention, and memory recall that contributes to the cognitive functions of learning, thinking, and memory.

In addition to memory acquisition, memory retention, and memory recall, the compositions including the combination of a source of choline, a source of magnesium, and a source of L-tryptophan may have a complementary mode of action in maintaining neuronal membrane fluidity, allowing for proper hormone and neurotransmitter function. This may further improve cognitive performance.

Additionally, in some embodiments, the compositions can be utilized to improve a cognitive impairment and/or brain dysfunction that may be associated with a neurodegenerative disease. While not wishing to be bound by theory, the combination of a source of choline, a source of magnesium, and a source of L-tryptophan shows anti-amyloidogenic properties and anti-inflammation properties, and are believed to reduce neuronal inflammation and clearance of amyloid β protein deposits that can lead to a cognitive impairment and/or brain dysfunction associated with neurodegenerative diseases or conditions such as Alzheimer's disease, Huntington's disease, Parkinson's disease, dementia, amyotrophic lateral sclerosis, stroke, and/or schizophrenia. By reducing this neuronal inflammation and clearing of the amyloid protein, cognitive function may be improved.

Furthermore, in some embodiments, the compositions of the present disclosure may improve a cognitive impairment and/or brain dysfunction associated with age-related cognitive decline or cognitive decline associated with a neurodegenerative disease by enhancing synaptic plasticity, which is controlled by N-methyl-D-aspartate receptor ("NMDAR"). While not wishing to be bound by theory, it is believed that deficits in synaptic plasticity and/or neuronal death can result in cognitive impairment and/or brain dysfunction. The compositions of the present disclosure are believed to restore the suboptimum level of NMDAR activity to optimum levels, even during weak stimulation, and, at the same time, prevent neuronal death from NMDAR over-activation. Consequently, NMDAR dependent hippocampal long term potentiation ("LTP") is believed to be enhanced, which is one key underlying molecular mechanism of learning and memory. Further, by preventing neuronal death, the neuronal loss associated with cognitive impairment and brain dysfunction can be avoided. In certain embodiments, the compositions of the present disclosure may further improve learning and memory in an individual by enhancing the NMDAR and 2-amino-3-(5-methyl-3-oxo-1,2-oxazol-4-yl) propanoic acid receptor (AMPAR) mediated hippocampal synoptic plasticity.

Although in some embodiments the methods of the present disclosure may be directed to subjects who have a neurodegenerative disease or condition, or a disease or condition related to a neurodegenerative disease or condition, the methods of the present disclosure as described herein are also intended in some embodiments to include the use of such methods in "at risk" subjects, including subjects unaffected by or not otherwise afflicted with any neurodegenerative disease or condition such as those described herein, for the purpose of preventing, minimizing, or delaying the development of such diseases or conditions over time. For such prevention purposes, the methods of the present disclosure preferably include repetitive, periodic administration of the compositions as described herein (i.e., administrations at "regular intervals"). Such preventive methods may be directed at adults, particularly older adults, who are susceptible to developing neurodegenerative diseases due to hereditary considerations, environmental considerations, and the like.

In certain embodiments, when the composition is a liquid composition, the serving may be 150 milliliters to 500 milliliters. In certain other embodiments, when the composition is a liquid, the serving is 237 milliliters (∼8 fl. oz.). In other embodiments, when the composition is a liquid, the serving is 177 milliliters to 414 milliliters (∼6 fl. oz. to ∼14 fl. oz.). In yet other embodiments, when the composition is a liquid, the serving is 207 milliliters to 296 milliliters (∼7 fl. oz. to ∼10 fl. oz.).

When in powder form, an amount of the powdered composition may be reconstituted into the form of a liquid composition (i.e., a "reconstituted composition"). In certain embodiments, the serving size of the reconstituted composition may be as defined for a liquid composition.

The composition is desirably administered to a subject in regular intervals. In various embodiments, administration at regular intervals includes daily administration, multiple daily administrations, or weekly administration(s). In further embodiments, the term "regular intervals" refers to administration 1-2 times per week, administration 1-3 times per week, administration 2-3 times per week, administration 1-4 times per week, administration 1-5 times per week, administration 2-5 times per week, administration 3-5 times per week, administration 1-6 times per week, administration 1-7 times per week, administration 2-6 times per week, administration 2-7 times per week, administration 1-2 times per day, administration 1-3 times per day, administration 1-4 times per day, administration 2-3 times per day, administration 2-4 times per day, administration 3-4 times per day, administration 2-5 times per day, administration 3-5 times per day, or administration 4-5 times per day. The subject desirably consumes the composition in an amount of at least one serving daily, and in some embodiments, may consume two, three, or even more servings per day.

Each serving is desirably administered as a single, undivided portion *(i.e.,* a "single serving"), although a serving may also be divided into a plurality of partial or divided portions (i.e., "a plurality of servings") to be consumed at two or more times during the day. For example, in certain embodiments, a serving is administered to a subject in multiple partial portions taken at regular intervals each day. The methods of the present disclosure include continuous day after day administration, as well as periodic or limited administration, although continuous day after day administration is generally desirable. The methods of the present disclosure are preferably applied on a daily basis, wherein the daily administration is maintained continuously for at least 3 days, including at least 5 days, including at least 1 month, including at least 6 weeks, including at least 8 weeks, including at least 2 months, including at least 6 months, desirably for at least about 18-24 months, desirably as a long term, continuous, daily, dietary supplement.

### EXAMPLE

The following example illustrates features of the compositions of the present disclosure as compared to various other compositions. The example is given solely for the purpose of illustration and is not to be construed as limitations of the present disclosure, as many variations thereof are possible without departing from the spirit and scope of the general inventive concepts. All exemplified amounts are weight percentages based upon the total weight of the composition, unless otherwise specified.

Four experimental diets were fed to experimental mice, including different combinations of ingredients. The diets were fed to mice, which were subsequently tested for cognitive abilities as described herein. At the beginning of the study, the mice were fifteen months old. The mice were fed one of the four diets for eight months, creating four experimental groups, with thirty mice per group. Group 1 was fed the control diet, which was a standard rodent diet. The diet fed to Group 2 was supplemented with hydroxytyrosol, PS-DHA, a carotenoid mix (lutein, lycopene, and beta-carotene), acetyl-L-carnitine, medium chain triglycerides, and a nucleotide mix (CMP, UMP, GMP, and AMP). The diet fed to Group 3 was the same as that fed to Group 2, except the diet fed to Group 3 was further supplemented with "Synergy-1," which is a combination of inulin, fructooligosaccharides, and L-fermentum (LC40). The diet fed to Group 4 was the same as that fed to Group 2, except the diet fed to Group 4 was further supplemented with a source of choline (glycerophosphocholine), a source of magnesium (magnesium threonate), and a source of L-tryptophan (L-tryptophan).

After seven months of being exclusively fed the experimental diets as described, a glass-covered transponder with unique identification codes (T) was injected subcutaneously in the interscapular area under isoflurane anesthesia. Twenty-four hours after transponder implantation, twelve mice from each experimental group (n=12) were placed into one IntelliCage apparatus for assessment of behavior. The IntelliCage apparatus is available from NewBehavior AG, Zurich, Switzerland. After a period of three weeks of adaptation, the mice were evaluated for cognitive performance.

The IntelliCage apparatus is a computer-based, fully automated testing apparatus that can be utilized for analysis of the spontaneous and learning behavior of radio frequency identification ("RFID")-tagged mice such as the mice of the experiments disclosed herein. The mice were tested according to a "shuttle protocol," which is further described herein. The shuttle protocol using an IntelliCage apparatus has been reported as a cognitive test related to executive function. *See, e.g.,* Endo et al., "Automated test of behavioral flexibility in mice using a behavioral sequencing task in IntelliCage," Behavioral Brain Research 221 (2011) 172-81. The executive brain function is a shorthand description of a set of cognitive processes that are responsible for appropriately organizing, performing, and maintaining goal-directed actions under ever-changing environmental contexts. The quality of life based on intellectual and mental integrity is largely dependent on this brain function, and its dysfunction is widely seen in people with aging-associated cognitive decline and various neuropsychological disorders.

A large standard plastic cage equipped with four triangular operant learning chambers (corners) that fit into each corner of the cage, RFID readers, and other types of sensors allows simultaneous monitoring of up to 16 transponder-tagged mice living in the same cage. Mice are allowed to enter the corner ("corner visit") through a relatively short, narrow tunnel that functions as an RFID antenna. In this unit, only one mouse can enter a corner at a time because of the limited size of the corner and tunnel.

In the inner space of the corner, mice can find two nose-poke holes with an infrared beam-break response detector. The "correct" nose-poke triggers the opening of a motorized access gate ("gate") to a water bottle nipple *(i.e.,* "rewarded"). An incorrect nose-poke triggers nothing *(i.e.,* "never-rewarded"). The time and duration of each behavioral event (corner visit, nose-poke, and lick), mouse identification, and corner identification are automatically recorded through RFID readers, infrared sensors, and "lickometers." Various protocols can be configured by the user in order to run different trials for testing several behaviors that may reflect different processes related to exploratory capabilities, learning, memory, and related cognitive skills in laboratory animals, particularly mice.

For the experiments at hand, the mice were tested according to the following "shuttle protocol." Mice were imposed to discriminate the rewarded and never-rewarded corners of the IntelliCage apparatus, and to shuttle between the two distantly positioned rewarded corners. Each mouse could open the gate and drink water for 4 seconds only from the rewarded corners, while they could never do so in the never-rewarded corners. Additionally, each of the two distantly positioned rewarded corners had two distinct states (i.e., "active" and "inactive") in a mutually exclusive manner between them. That is, there is always one active rewarded corner, one inactive rewarded corner, and two never-rewarded corners at a time. The gates could be opened by a nose-poke action only in an active rewarded corner. When a mouse did a nose-poke in the active rewarded corner and was presented a reward (i.e., water), the corner became inactive. At the same time, the inactive rewarded corner became the active rewarded corner. Thus, the mice had to shuttle between the two distantly positioned rewarded corners. All the corner assignments were balanced within a group in the cage so that no specific corner would receive more traffic than others.

From time to time, diagonal spatial patterns of rewarded and never-rewarded corners were reversely changed. Thus, the mice had to learn to switch their shuttling behavior between the two diagonal spatial patterns.

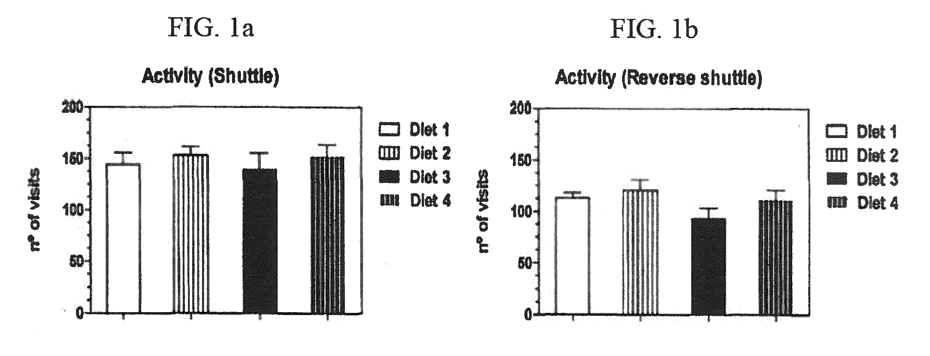

The activity of the mice, measured as total number of visits to the different corners, was approximately the same among groups during both cognitive tests ("shuttle" and "reverse shuttle"). This data is illustrated in FIGs. 1 a ("shuttle") and 1 b ("reverse shuttle").

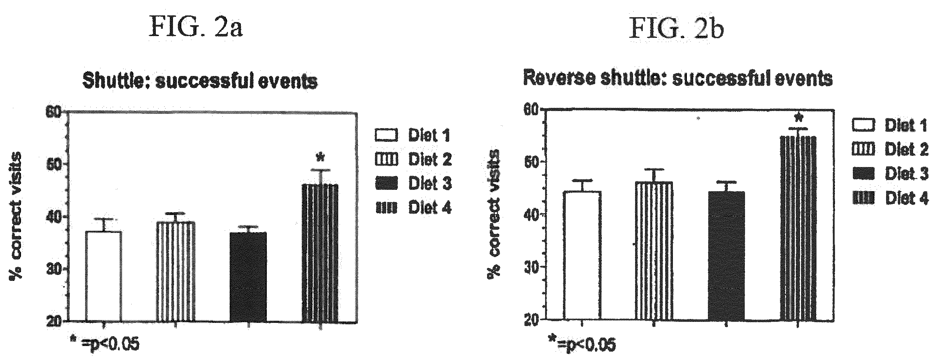

With respect to all Groups and as illustrated in FIG. 2a, the percentage of correct visits for the "shuttle" tests was significantly higher in Group 4, which was fed the diet that was supplemented with the source of choline, the source of magnesium, and the source of L-tryptophan. FIG. 2b illustrates that the percentage of correct visits was maintained even in the "reverse shuttle" tests. These results indicate that, in addition to better performance than the other groups, Group 4 was able to be more flexible in cognition under ever-changing environmental conditions.

To the extent that the terms "include," "includes," or "including" are used in the specification or the claims, they are intended to be inclusive in a manner similar to the term "comprising" as that term is interpreted when employed as a transitional word in a claim. Furthermore, to the extent that the term "or" is employed (*e.g.,* A or B), it is intended to mean "A or B or both A and B." When the applicants intend to indicate "only A or B but not both," then the term "only A or B but not both" will be employed. Thus, use of the term "or" herein is the inclusive, and not the exclusive use. Also, to the extent that the terms "in" or "into" are used in the specification or the claims, it is intended to additionally mean "on" or "onto."

The general inventive concepts have been illustrated, at least in part, by describing various exemplary embodiments thereof. While these exemplary embodiments have been described in considerable detail, it is not the Applicant's intent to restrict or in any way limit the scope of the appended claims to such detail. Furthermore, the various inventive concepts may be utilized in combination with one another (e.g., one or more of the first, second, third, fourth, etc., exemplary embodiments may be utilized in combination with each other). Additionally, any particular element recited as relating to a particularly disclosed embodiment should be interpreted as available for use with all disclosed embodiments, unless incorporation of the particular element would be contradictory to the express terms of the embodiment. Additional advantages and modifications will be readily apparent to those skilled in the art. Therefore, the disclosure, in its broader aspects, is not limited to the specific details presented therein, any representative apparatus, or the illustrative example shown and described. Accordingly, departures may be made from such details without departing from the spirit or scope of the general inventive concepts.

## Claims

1. A composition suitable for maintaining or improving cognition in a subject, the composition comprising: a source of choline in an amount sufficient to provide 0.4 to 8 grams of choline per day; a source of magnesium in an amount sufficient to provide 0.2 to 3 grams of magnesium per day; and a source of L-tryptophan.

2. A composition suitable for maintaining or improving cognition in a subject, the composition comprising: 0.1 to 40 weight percent of a source of choline; 0.1 to 30 weight percent of a source of magnesium; and a source of L-tryptophan; wherein the weight percentages are of the total solids of the composition.

3. The composition of claim 1 or 2, wherein the source of choline is selected from the group consisting of choline, phosphocholine, phosphatidyl-choline, glycerophosphocholine, and combinations thereof.

4. The composition of claim 3, wherein the source of choline is glycerophosphocholine.

5. The composition of claim 4, wherein the source of magnesium is magnesium threonate.

6. The composition of claim 1, further comprising a source of threonate in an amount sufficient to provide 0.2 to 3 grams of threonate per day.

7. The composition of claim 6, wherein the magnesium source is magnesium threonate and the threonate source is magnesium threonate.

8. The composition of claim 1 or 2, wherein the source of magnesium is selected from the group consisting of magnesium citrate, magnesium chloride, magnesium threonate, and combinations thereof.

9. The composition of claim 1 or 2, further comprising at least one large neutral amino acid, such that a weight ratio of L-tryptophan-to-total large neutral amino acids in the composition ranges from 0.2 to 0.5.

10. The composition of claim 1, wherein the composition is a nutritional composition further comprising at least one of a protein, a carbohydrate, and a lipid.

11. The composition of claim 10, wherein the nutritional composition takes the form of a powder, a solid, a semi-solid, a liquid, or a semi-liquid.

12. The composition of claim 10, wherein the nutritional composition takes the form of a liquid or semi-liquid and further comprises water.

13. The composition of claim 10, wherein the nutritional composition takes the form of a nutritional bar.

14. The composition of claim 10, further comprising at least one of a vitamin and a mineral.

15. A composition for use in maintaining or improving cognition in a subject, the composition comprising a source of choline, a source of magnesium, and a source of L-tryptophan, wherein an effective amount of the composition maintains or stimulates brain function in the subject and thereby maintains or improves cognition of the subject.

16. A composition according to claim 15, wherein the source of choline is selected from the group consisting of choline, phosphocholine, phosphatidyl-choline, glycerophosphocholine, and combinations thereof.

17. A composition according to claim 16, wherein the source of choline is glycerophosphocholine.

18. A composition according to any one of claims 15 to 17, wherein the source of magnesium is selected from the group consisting of magnesium citrate, magnesium chloride, magnesium threonate, and combinations thereof.

19. A composition according to any one of claims 15 to 17, wherein the composition further comprises a source of threonate.

20. A composition according to claim 19, wherein the source of magnesium is magnesium threonate and the source of threonate is magnesium threonate.

21. A composition according to any one of claims 15 to 17, wherein the composition is administered orally.

22. A composition according to any one of claims 15 to 17, wherein the composition is administered in regular intervals.

23. A composition according to claim 22, wherein the regular intervals are selected from the group consisting of: daily administration; multiple daily administrations; and weekly administrations.

24. A composition according to claim 22, wherein the effective amount is administered in a single serving per day.

25. A composition according to claim 22, wherein the effective amount is administered in a plurality of servings per day.

26. A composition according to any one of claims 15 to 17, wherein the composition takes the form of a powder, a solid, a semi-solid, a liquid, or a semi-liquid.

27. A composition according to any one of claims 15 to 17, wherein the subject is a human.

28. A composition as defined in any one of claims 1 to 15 for use in the treatment of the human or animal body by therapy.

29. A composition according to claim 28 for maintaining or improving cognition.

## Amended claims

### Amended claims in accordance with Rule 137(2) EPC.

1. A composition suitable for maintaining or improving cognition in a subject, the composition comprising: a source of choline in an amount sufficient to provide 0.4 to 8 grams of choline per day; a source of magnesium in an amount sufficient to provide 0.2 to 3 grams of magnesium per day; a source of threonate, and a source of L-tryptophan.

2. A composition suitable for maintaining or improving cognition in a subject, the composition comprising: 0.1 to 40 weight percent of a source of choline; 0.1 to 30 weight percent of magnesium threonate; and a source of L-tryptophan; wherein the weight percentages are of the total solids of the composition.

3. The composition of claim 1 or 2, wherein the source of choline is selected from the group consisting of choline, phosphocholine, phosphatidyl-choline, glycerophosphocholine, and combinations thereof.

4. The composition of claim 3, wherein the source of choline is glycerophosphocholine.

5. The composition of claim 1, wherein the source of magnesium is magnesium threonate.

6. The composition of claim 1, wherein the source of threonate is in an amount sufficient to provide 0.2 to 3 grams of threonate per day.

7. The composition of claim 6, wherein the magnesium source is magnesium threonate and the threonate source is magnesium threonate.

8. The composition of claim 1, wherein the source of magnesium is selected from the group consisting of magnesium citrate, magnesium chloride, magnesium threonate, and combinations thereof.

9. The composition of claim 1 or 2, further comprising at least one large neutral amino acid, such that a weight ratio of L-tryptophan-to-total large neutral amino acids in the composition ranges from 0.2 to 0.5.

10. The composition of claim 1, wherein the composition is a nutritional composition further comprising at least one of a protein, a carbohydrate, and a lipid.

11. The composition of claim 10, wherein the nutritional composition takes the form of a powder, a solid, a semi-solid, a liquid, or a semi-liquid.

12. The composition of claim 10, wherein the nutritional composition takes the form of a liquid or semi-liquid and further comprises water.

13. The composition of claim 10, wherein the nutritional composition takes the form of a nutritional bar.

14. The composition of claim 10, further comprising at least one of a vitamin and a mineral.

15. A composition for use in the treatment or prevention of a neurodegenerative disease or condition, the composition comprising a source of choline, a source of magnesium, and a source of L-tryptophan, wherein an effective amount of the composition maintains or stimulates brain function in the subject and thereby maintains or improves cognition of the subject.

16. A composition according to claim 15, wherein the source of choline is selected from the group consisting of choline, phosphocholine, phosphatidyl-choline, glycerophosphocholine, and combinations thereof.

17. A composition according to claim 16, wherein the source of choline is glycerophosphocholine.

18. A composition according to any one of claims 15 to 17, wherein the source of magnesium is selected from the group consisting of magnesium citrate, magnesium chloride, magnesium threonate, and combinations thereof.

19. A composition according to any one of claims 15 to 17, wherein the composition further comprises a source of threonate.

20. A composition according to claim 19, wherein the source of magnesium is magnesium threonate and the source of threonate is magnesium threonate.

21. A composition according to any one of claims 15 to 17, wherein the composition is administered orally.

22. A composition according to any one of claims 15 to 17, wherein the composition is administered in regular intervals.

23. A composition according to claim 22, wherein the regular intervals are selected from the group consisting of: daily administration; multiple daily administrations; and weekly administrations.

24. A composition according to claim 22, wherein the effective amount is administered in a single serving per day.

25. A composition according to claim 22, wherein the effective amount is administered in a plurality of servings per day.

26. A composition according to any one of claims 15 to 17, wherein the composition takes the form of a powder, a solid, a semi-solid, a liquid, or a semi-liquid.

27. A composition according to any one of claims 15 to 17, wherein the subject is a human.

28. A composition as defined in any one of claims 1 to 15 for use in the treatment of the human or animal body by therapy.

29. A composition according to claim 28 for maintaining or improving cognition.
